# EUROPEAN PATENT SPECIFICATION

(11) **EP 1 108 722 B1**
(45) Date of publication and mention of the grant of the patent: **27.04.2005**
(21) Application number: 00204456.8
(22) Date of filing: 12.12.2000
(51) Int. Cl.: C07D 471/04

(54) **Trazodone hydrochloride and process for its preparation**
Trazodon-Hydrochlorid und Verfahren zu seiner Herstellung
Chlorhydrate de Trazodone et procédé pour sa préparation

(30) Priority: 16.12.1999 IT MI992598
(43) Date of publication of application: 20.06.2001
(73) Proprietor: AZIENDE CHIMICHE RIUNITE ANGELINI FRANCESCO A.C.R.A.F. S.p.A., 00181 Roma (IT)
(72) Inventor: Segnalini, Franca, 04100 Latina (IT)
(74) Representative: Marchi, Massimo

(56) References cited:
- FR-A- 2 318 871
- GB-A- 1 117 068

## Description

This invention relates to a trazodone hydrochloride (i.e. 2-[3-[4-(3-chlorophenyl)-1-piperazinyl)propyl]-1,2,4-triazolo[4,3-a]pyridin-3(2H)-one hydrochloride) having a controlled particle size and a process for controlling the particle size of the trazodone hydrochloride at will.

In accordance with the known art trazodone hydrochloride is prepared using ethereal hydrochloric acid (Italian Patent 1 066 857, GB 1 117 068).

This process however cannot be used on an industrial scale because it involves the use of large quantities of gaseous hydrochloric acid.

The Applicant therefore attempted to use an aqueous solution of hydrochloric acid. However, to avoid relevant reductions in yield caused by the solubility of trazodone hydrochloride in water, the Applicant used the least possible amount of water in the reaction mixture. He therefore carried out a number of production runs on an industrial scale in which an aqueous solution of 12 N hydrochloric acid was added to an organic solution of trazodone base.

However, this method yields a trazodone hydrochloride which has such a fine particle size that it does not sediment out from the body of the solution and is difficult to filter off. Also, when in the state of a dry powder, it has an average particle size of a few microns, retains large quantities of solvents and has an apparent density which is so low as to give rise to numerous disadvantages during the production of pharmaceutical formulations, particularly solid formulations, such as tablets and capsules.

Now it has surprisingly been found that if a more dilute aqueous solution of hydrochloric acid, for example 6 N instead of 12 N, is added to the organic solution of trazodone base, a trazodone hydrochloride having an average particle size of approximately 50 µm is obtained.

Even more surprisingly it has been found that an even larger particle size is obtained when part of the same identical amount of water is added to a solution of trazodone base in organic solvents even before the aqueous solution of hydrochloric acid is added.

Furthermore, it has been found that the addition of water prior to acidification has the further advantage that it reduces the amount of residual reaction solvent in the final trazodone hydrochloride (see Example 1) in comparison with when water is added only in the form of an aqueous solution of hydrochloric acid.

In the present description and claims the term "residual reaction solvent" is used to mean organic solvents from either the preparation of the trazodone base or the preparation of the trazodone hydrochloride.

In turn the expression "average particle size" is used to indicate that 50% of the particles of the powder in question have a dimension less than the value indicated when measured using a laser granulometer with dispersion in liquid. For example, an average particle size ≥ 50 µm indicates that 50% of the powder particles have a size greater than or equal to 50 µm.

Typically trazodone hydrochloride according to this invention has an average particle size ≥ 10 µm.

Preferably the trazodone hydrochloride according to this invention has an average particle size ≥ 30 µm. Even more preferably it has an average particle size ≥ 50 µm. Advantageously the trazodone hydrochloride according to this invention has an average particle size ≤ 1000 µm, preferably ≤ 500 µm, and even more preferably ≤ 300 µm.

In an aspect this invention relates to a process for controlling the particle size of trazodone hydrochloride according to claim 1

Preferably the water is added in two steps, first alone and then in the form of an aqueous solution of hydrochloric acid.

A person skilled in the art will not have any difficulty in preparing trazodone hydrochloride having the desired particle size by suitably metering the amount of water added before or together with the hydrochloric acid.

Typical examples of organic solvents which can be used in the aforesaid process are polar solvents which are miscible or partly miscible with water such as for example esters, ketones, or aliphatic or cycloaliphatic alcohols having a low molecular weight and preferably from 1 to 6 carbon atoms, such as ethyl acetate, acetone, methanol, ethanol and hexanol.

Preferably the ratio between the trazodone base and the organic solvent is of from 1:3 to 1:10 (w/v) while the ratio between the trazodone base and water is of from 1:0.13 to 1:0.5 (w/v).

The following Examples intend to illustrate this invention without however limiting it in any way.

### EXAMPLE 1

### Trazodone hydrochloride [average particle size = 127 µm; apparent density = 0.79 g/cm³]

Trazodone base (47.0 g) prepared in accordance with the method described in Italian Patent No. 1 066 857 lodged by the Applicant and containing from 1 to 40% of isobutanol was dissolved in acetone to a volume of 270 ml. Water (10.1 ml) was added to the thus obtained acetone solution and the whole was heated to 50°C under stirring. Then an aqueous solution of hydrochloric acid (12N, 9.5 ml) was added over 20 minutes while maintaining the temperature of from 48 to 52°C, under stirring, up to a pH 3-4.

The thus obtained solution was kept at 50°C for additional 15-30 minutes and then cooled slowly. When the temperature of 5°C had been reached, the thus cooled solution was filtered on a Buchner filter, washed with 2 x 50 ml of acetone and the solvent was removed under vacuum at 80°C for 16 hours.

In this way 40.3 g of the desired product having the following characteristics were obtained:

| | |
|---|---|
| average particle size | 127 µm |
| final acetone content | 178 ppm |
| isobutanol content | 32 ppm |

The value of the average particle size was tested by means of a laser granulometer from the Malvern company, with liquid dispersion, type MasterSizer Microplus.

### EXAMPLE 2

### Trazodone hydrochloride [average particle size = 47 µm; apparent density = 0.58 g/cm³]

The procedure was the same as in the previous Example 1, except that not only water but an aqueous solution of hydrochloric acid (6N, 19.3 ml) was added to the initial acetone solution of trazodone base.

In this way 40 g of the desired product having the following characteristics were obtained:

| | |
|---|---|
| average particle size | 47 µm |
| final acetone content | 656 ppm |
| isobutanol content | 71 ppm |

The value of the average particle size was tested by means of a laser granulometer from the Malvern company, with liquid dispersion, type MasterSizer Microplus.

## Claims

1. A process for controlling the particle size of trazodone hydrochloride, comprising dissolving trazodone base in an organic solvent and adding water and hydrochloric acid to that solution, **characterized in that** the amount of water is of at least 0.15 parts per each part by weight of trazodone base.

2. A process according to claim 1, **characterized in that** water is added in two steps, first alone and then in the form of an aqueous solution of hydrochloric acid.

3. A process according to claim 1 or 2, **characterized in that** the said organic solvent is a polar solvent which is miscible or partly miscible with water.

4. A process according to any one of claims 1 to 3, **characterized in that** the ratio between the trazodone base and the organic solvent is of from 1:3 to 1:10 (w/v) while the ratio between the trazodone base and water is of from 1:0.13 to 1:0.5 (w/v).

## Patentansprüche

1. Verfahren zur Steuerung der Teilchengrösse von Trazodon-hydrochlorid, das das Auflösen von Trazodonbase in einem organischen Lösungsmittel und die Zugabe von Wasser und Salzsäure zu der Lösung umfasst, **dadurch gekennzeichnet, dass** die Menge an Wasser mindestens 0,15 Teile pro Gew.-Teil Trazodonbase beträgt.

2. Verfahren gemäss Anspruch 1, **dadurch gekennzeichnet, dass** das Wasser in zwei Schritten zugegeben wird, zunächst alleine und dann in Form einer wässrigen Salzsäurelösung.

3. Verfahren gemäss Anspruch 1 oder 2, **dadurch gekennzeichnet, dass** das organische Lösungsmittel ein polares Lösungsmittel ist, das mit Wasser mischbar oder teilweise mischbar ist.

4. Verfahren gemäss mindestens einem der Ansprüche 1 bis 3, **dadurch gekennzeichnet, dass** das Verhältnis zwischen Trazodonbase und organischem Lösungsmittel 1:3-1:10(G/V) beträgt, während das Verhältnis zwischen Trazodonbase und Wasser 1:0,13-1:0,5 (G/V) beträgt.

## Revendications

1. Procédé pour contrôler la taille particulaire du chlorhydrate de trazodone, comprenant la dissolution de la trazodone base dans un solvant organique et l'addition d'eau et d'acide chlorhydrique à cette solution, **caractérisé en ce que** la quantité d'eau est au moins de 0,15 partie pour chaque partie en masse de trazodone base.

2. Procédé selon la revendication 1, **caractérisé en ce que** l'eau est ajoutée en deux étapes, tout d'abord seule et ensuite sous la forme d'une solution aqueuse d'acide chlorhydrique.

3. Procédé selon la revendication 1 ou 2, **caractérisé en ce que** ledit solvant organique est un solvant polaire, qui est miscible ou partiellement miscible à l'eau.

4. Procédé selon l'une quelconque des revendications 1 à 3, **caractérisé en ce que** le rapport entre la trazodone base et le solvant organique est compris entre 1:3 et 1:10 (m/v) alors que le rapport entre la trazodone base et l'eau est compris entre 1:0,13 et 1:0,5 (m/v).
